(19) Europäisches Patentamt
European Patent Office
Office européen des brevets

(11) Publication number: 0 116 432 A2

# (12) EUROPEAN PATENT APPLICATION

(21) Application number: 84300560.4

(22) Date of filing: 30.01.84

(51) Int. Cl.³: C 07 D 205/08
C 07 D 403/04, C 07 D 413/04
//C07D207/26

(30) Priority: 02.02.83 US 463101

(43) Date of publication of application: 22.08.84 Bulletin 84/34

(84) Designated Contracting States: BE CH DE FR GB IT LI LU NL SE

(71) Applicant: THE UNIVERSITY OF NOTRE DAME du LAC
Notre Dame Indiana 46556(US)

(72) Inventor: Miller, Marvin Joseph
17885 Tally Ho Drive
South Bend Indiana 46635(US)

(74) Representative: Hudson, Christopher Mark et al,
Erl Wood Manor
Windlesham Surrey GU20, 6PH(GB)

## (54) Improvements in or relating to N-(substituted-methyl)-azetidin-2-ones.

(57) N-(Mono- or disubstituted methyl)-2-azetidinones, produced by cyclizing β-hydroxy or β-halo substituted acid sec-amides in which the amide nitrogen is subsituted with a mono- or di-substituted methyl group having activating substituents, are useful intermediates for the production of antibiotics. The compounds have the formula

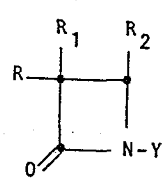 (I)

in which R is hydrogen, $C_1$-$C_4$ alkoxy, amino, protected-amino, acylamino, diacylamino, $C_1$-$c_4$ alkyl, or $C_1$-$C_4$ alkyl sustituted by hydroxy, $C_1$-$C_4$ alkoxy, halogen, amino, protected-amino, carboxy, or protected carboxy;

$R_1$ is hydrogen, hydroxy, $C_1$-$C_4$ alkoxy, halogen, or $C_1$-$C_4$ alkyl;

$R_2$ is hydrogen, phenyl, substituted phenyl, carboxy, $C_1$-$C_4$ alkoxycarbonyl, protected carboxy, sulfydryl, $C_1$-$C_4$ alkylthio, $C_2$-$C_4$ alkanoylthio, halogen, $C_1$-$C_4$ alkoxy, $C_1$-$C_4$ alkyl, or $C_1$-$C_4$ alkyl substituted by hydroxy, $C_1$-$C_4$ alkoxy, halogen, amino, protected amino, carboxy, or protected carboxy;

Y is a substituted methyl group of the formulae

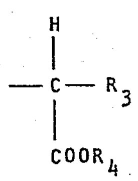 , 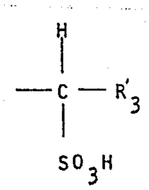 , or 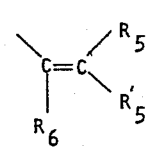

in which $R_3$ or $R_3'$ is hydrogen, $C_1$-$C_4$ alkoxycarbonyl, protected carboxy, carboxy, $C_1$-$C_4$ alkylsulfonyl, arylsulfonyl, $C_1$-$C_4$ alkylsulfinyl, arylsulfinyl, $C_1$-$C_4$ alkanoyl, aroyl, $C_1$-$C_4$ alkyl, cyano, vinyl, or ethinyl;

$R_4$ is hydrogen, $C_1$-$C_4$ alkyl, or a carboxy protecting group;

$R_5$ and $R_5'$, independently are $C_1$-$C_4$ alkyl, $C_1$-$C_4$ alkoxycarbonyl, carboxy, protected-carboxy, or phenyl; and,

$R_6$ is hydrogen, $C_1$-$C_4$ alkyl, $C_1$-$C_4$ alkoxy-carbonyl, protected-carboxy, or phenyl; or an addition salt thereof.

EP 0 116 432 A2

# IMPROVEMENTS IN OR RELATING TO N-(SUBSTITUTED-METHYL)-AZETIDIN-2-ONES

This invention relates to monocyclic β-lactam compounds and to a process for the preparation thereof. In particular, this invention relates to N-(mono or di-substituted-methyl)-azetidin-2-ones and related compounds and to a method for the preparation thereof which comprises the cyclization of a β-hydroxy or β-halo N-mono or di-substituted-methyl amide. The compounds of the invention are useful for the preparation of monocyclic β-lactam antibiotics, β-lactamase inhibitors, and bicyclic β-lactam antibiotic compounds.

The class of β-lactam antibiotics includes the well-known penicillin and cephalosporin antibiotics. New β-lactam antibiotics have been discovered recently, for example, thienamycin, nocardicin, clavulanic acid, isoclavulanic acid, and monobactam (Belgium Patent No. 887,428). Because of the importance of the β-lactam antibiotics, a need exists for improved methods for their production. Consequently, considerable research is being conducted to develop simple and economic routes of synthesis.

This invention provides monocyclic β-lactam compounds of general Formula (I):

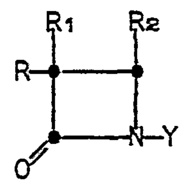

(I)

in which R is preferably an amino or an acylamino group, $R_1$ and $R_2$ represent substituent groups, for example, lower alkyl, lower alkoxy and substituted alkyl groups, and Y represents a mono or di-substituted-methyl group wherein at least one of the substituent groups is an electron withdrawing group, or Y is a substituted vinyl group wherein one of the substituents is likewise an electron withdrawing group. This invention also provides a process for the preparation of the compounds of the formula 1 which comprises the cyclization of a β-hydroxy or β-halo-substituted amide represented by Formula (II):

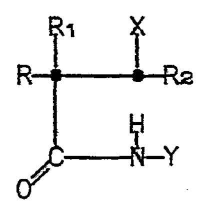

(II)

in which Y is a mono or di-substituted-methyl group in which either or both of the substituents are electron withdrawing groups such as the sulfo group or an esterified carboxy group, and X is hydroxy or halo. The process may be performed with a compound of Formula (II) in which X is hydroxy with the complex formed with a dialkylazodicarboxylate and a phosphorus compound selected from triphenylphosphine, triphenylphosphite,

diphenyl phenylphosphonate, and phenyldiphenylphosphinoate; and when X is halo, such as chloro or bromo, cyclization is carried out with a lithium dialkylamide.

The compounds of the invention are useful in the synthesis of β-lactam antibiotics and β-lactamase inhibitors.

In accordance with the invention, therefore, compounds of Formula (I):

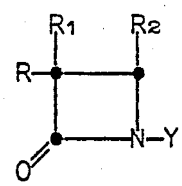

(I)

in which R is hydrogen, $C_1$-$C_4$ alkoxy, amino, protected amino, acylamino, diacylamino, $C_1$-$C_4$ alkyl, or $C_1$-$C_4$ alkyl substituted by hydroxy, $C_1$-$C_4$ alkoxy, halogen, amino, protected-amino, carboxy, or protected carboxy;

$R_1$ is hydrogen, hydroxy, $C_1$-$C_4$ alkoxy, halogen, or $C_1$-$C_4$ alkyl;

$R_2$ is hydrogen, phenyl, substituted phenyl, carboxy, $C_1$-$C_4$ alkoxycarbonyl, protected carboxy, sulfydryl, $C_1$-$C_4$ alkylthio, $C_2$-$C_4$ alkanoylthio, halogen, $C_1$-$C_4$ alkoxy, $C_1$-$C_4$ alkyl, or $C_1$-$C_4$ alkyl substituted by hydroxy, $C_1$-$C_4$ alkoxy, halogen, amino, protected amino, carboxy, or protected carboxy;

Y is a substituted methyl group of the formulae

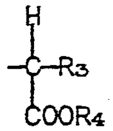 , 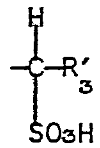 , or 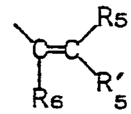

in which $R_3$ or $R_3'$ is hydrogen, $C_1$-$C_4$ alkoxycarbonyl, protected-carboxy, carboxy, $C_1$-$C_4$ alkylsulfonyl, arylsulfonyl, $C_1$-$C_4$ alkylsulfinyl, arylsulfinyl, $C_1$-$C_4$ alkanoyl, aroyl, $C_1$-$C_4$ alkyl, cyano, vinyl, or ethinyl;

$R_4$ is hydrogen, $C_1$-$C_4$ alkyl, or a carboxy protecting group;

$R_5$ and $R_5'$, independently, are $C_1$-$C_4$ alkyl, $C_1$-$C_4$ alkoxycarbonyl, carboxy, protected-carboxy, or phenyl; and,

$R_6$ is hydrogen, $C_1$-$C_4$ alkyl, $C_1$-$C_4$ alkoxycarbonyl, protected-carboxy, or phenyl; or an acid addition salt thereof, are useful in the preparation of β-lactam antibiotics.

In the above definitions, $C_1$-$C_4$ alkoxy refers to methoxy, ethoxy, n-propoxy, iso-propoxy, n-butyloxy, and t-butyloxy; $C_1$-$C_4$ alkyl refers to the straight and branched chain $C_1$-$C_4$ alkyl groups such as methyl, ethyl, n-propyl, iso-propyl, n-butyl, sec-butyl, and t-butyl; halogen refers to fluoro, chloro, bromo, and iodo; $C_1$-$C_4$ alkyl substituted by hydroxy, $C_1$-$C_4$ alkoxy, halogen, amino, protected amino, carboxy, or protected carboxy refers to the hydroxy-substituted lower alkyl groups, for example, hydroxymethyl, 2-hydroxyethyl, 1-hydroxyethyl, 3-hydroxypropyl, or 2-hydroxybutyl; $C_1$-$C_4$ alkyl substituted by $C_1$-$C_4$ alkoxy refer to such

groups as methoxymethyl, ethoxymethyl, 2-methoxyethyl, 2-ethoxyethyl, and t-butyloxymethyl; halo-substituted $C_1$-$C_4$ alkyl refers to groups such as iodomethyl, bromomethyl, fluoromethyl, 2-chloroethyl, 2-bromoethyl, and 3-chloropropyl; $C_1$-$C_4$ alkyl substituted by amino refers to the lower aminoalkyl radicals such as aminomethyl, 2-aminoethyl, 3-aminopropyl, 2-aminopropyl, and 3-aminobutyl; $C_1$-$C_4$ alkyl substituted by protected amino refers to amino-substituted $C_1$-$C_4$ alkyl groups such as those mentioned above in which the amino group is protected by a conventional amino protecting group such as an alkoxycarbonyl or cycloalkoxycarbonyl group, a benzyloxycarbonyl or substituted benzyloxycarbonyl group, an enamine protecting group, or a cleavable acyl group such as chloroacetyl, or dichloroacetyl; carboxy-substituted $C_1$-$C_4$ alkyl refers to carboxymethyl, 2-carboxyethyl, 3-carboxypropyl, 2-carboxypropyl, or 4-carboxybutyl; while $C_1$-$C_4$ alkyl substituted by protected carboxy refers to carboxy-substituted lower alkyl groups such as those mentioned above in which the carboxy group is protected by conventional carboxy-protecting groups such as benzyl or substituted benzyl, diphenylmethyl or substituted diphenylmethyl, substituted alkyl such as trichloroethyl, 2-iodoethyl, phenacyl, trialkylsilyl, and t-butyl; $C_1$-$C_4$ alkoxycarbonyl refers to methoxycarbonyl, ethoxycarbonyl, n-propoxycarbonyl, t-butoxycarbonyl, n-butoxycarbonyl, and other lower alkoxycarbonyl groups.

The term sulfhydryl refers to the -SH group while $C_1$-$C_4$ alkylthio refers to alkyl-S-groups such as methylthio, ethylthio, propylthio and butylthio.

"$C_2$-$C_4$ Alkanoylthio" refers to $alk-\overset{O}{\overset{\|}{C}}-S$ groups such as acetylthio or propionylthio.

The term "substituted phenyl" refers to phenyl substituted by $C_1$-$C_4$ alkyl, $C_1$-$C_4$ alkoxy, carboxy, protected carboxy, hydroxy, halogen, or cyano.

The term "$C_1$-$C_4$ alkanoyl" refers to groups such as formyl, acetyl, propionyl, or butyryl.

Representative of the 3-acylamino groups (Formula (I), R is acylamino) are those in which the acyl groups are derived from carboxylic acids, in particular the acyl groups found in the 7-position of cephalosporin and the 6-position of penicillin antibiotics. In particular, the acyl group of the acylamino group, $R_1$ is $C_1$-$C_5$ alkanoyl, substituted $C_2$-$C_5$ alkanoyl substituted by halogen, cyano, or hydroxy; an arylacetyl or heteroarylacetyl group represented by the Formula

$$R^{\circ}-\underset{Q}{\underset{|}{CH}}-\overset{O}{\overset{\|}{C}}-$$

in which R° is thienyl, benzothienyl, furyl, benzofuryl, thiazolyl, oxazolyl, isoxazolyl, isothiazolyl, pyrazolyl, thiadiazolyl, oxadiazolyl, pyridyl, or these heterocyclic rings substituted by $C_1$-$C_4$ alkyl, amino, protected amino, or hydroxy; cyclohexadienyl, naphthyl,

phenyl, or a substituted phenyl group represented by the Formula

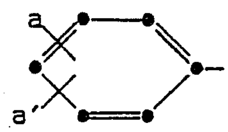

,

in which a and a', independently, are hydrogen, halogen, hydroxy, $C_1$-$C_4$ alkyl, $C_1$-$C_4$ alkoxy, amino, aminomethyl, methylsulfonylamino, hydroxymethyl, trifluoromethyl, carboxy, protected carboxy, carboxymethyl, or protected carboxymethyl; Q is hydrogen, hydroxy, $C_1$-$C_4$ alkanoyloxy, carboxy, protected carboxy, sulfo(-$SO_3$H), amino, protected amino, or a substituted amino group represented by the Formula

```
 H O x O
 ' " ' "
-N-C-N-C-R'
```

in which R' is furyl, thienyl, phenyl, halophenyl, nitrophenyl, styryl, halostyryl, nitrostyryl; or a group of the Formula

```
 y
 '
-N-R''
```

in which R'' is hydrogen, $C_1$-$C_4$ alkyl, benzyl, $C_2$-$C_5$ alkanoyl, or $C_1$-$C_3$ alkylsulfonyl; and x and y, when taken separately, are hydrogen or $C_1$-$C_4$ alkyl, and when taken together form a 5- or 6-membered ring represented by the Formula

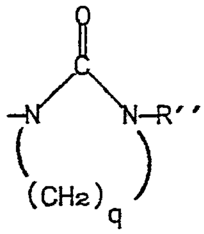

in which R'' has the same meanings as defined earlier and q is 2 or 3; or

Q is a substituted amino group of the Formula

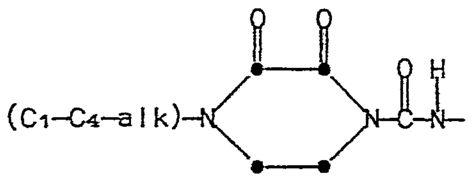

;

or Q is a benzamido group represented by the Formula

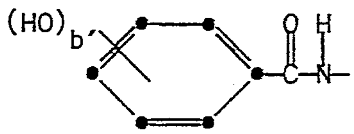

in which b' is an integer of from 1-3;

or R is an acylamino group of the formula

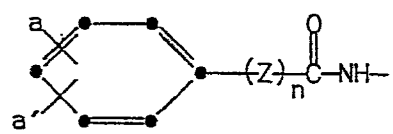

in which a and a' have the same meanings as defined earlier, Z is O or S, and n is 0 or 1;

or an oximino-substituted acylamino group of the Formula

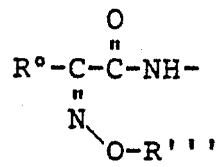

in which R° is as defined earlier, and R''' is hydrogen, $C_1$-$C_4$ alkyl, or a carboxy-substituted alkyl or cycloalkyl group of the Formula

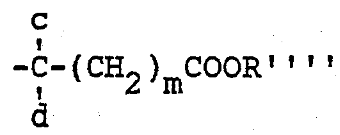

in which m is 0-3, and c and d when taken separately are, independently, hydrogen or $C_1$-$C_3$ alkyl, and when taken together with the carbon atom to which they are bonded form a 3 to 6-membered carbocyclic ring; and wherein R'''' is hydrogen, $C_1$-$C_4$ alkyl, or a carboxy-protecting ester forming group.

When R in Formula (I) is a diacylamino group, R represents a group of the formula

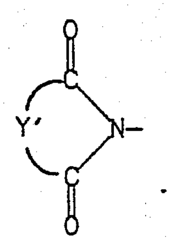

in which Y' is $C_1$-$C_3$ alkylene or o-phenylene. Examples of diacylamino groups represented by the above formula are phthalimido, succinimide, and maleimido.

When R in Formula (I) is a protected-amino group, R is an amino group substituted by a conventional amino-protecting group used in the art for the temporary protection of an amino group. Examples of such conventional protecting groups are the alkoxycarbonyl, substituted alkoxycarbonyl, cycloalkoxycarbonyl, bicyclicalkoxycarbonyl, alkenyloxycarbonyl, and arylalkoxycarbonyl groups. Examples of these protecting groups are ethoxycarbonyl, cyclopentyloxycarbonyl, cyclohexyloxycarbonyl, 2,2,2-trichloroethoxycarbonyl, allyloxycarbonyl, 1-adamantyloxycarbonyl, t-butyloxycarbonyl, t-amyloxycarbonyl, benzyloxycarbonyl (Cbz), p-nitrobenzyloxycarbonyl, p-methoxybenzyloxycarbonyl, or diphenylmethoxycarbonyl. Other protecting groups are the trialkylsilyl-protecting groups such as trimethylsilyl, and the bis-trialkylsilyl-protecting groups such as N,N-bis-trimethylsilyl. Other convenient amino-protecting groups are the phthaloyl group and the formyl group. An especially preferred amino-protecting group of this invention is the 4,5-diphenyl-4-oxazolin-2-one group formed with the amino group and 1,2-diphenylvinylene carbonate (J. C. Sheehan, et al., J. Org. Chem., 18, No. 17, 3034-3040 [1973]).

The amino-protecting group serves as a blocking group for the temporary protection of the amino group in the 3-position of the azetidinone ring. Such protecting groups are known for their stability under the reaction conditions used but are easily removable by conventional means afterwards. In particular, the 3-amino group is protected to prevent competitive amide formation reactions.

"Carboxy-protecting" group, as employed in the definition of the compounds represented by Formula (I), refers to conventional carboxylic acid-protecting ester groups commonly used in the β-lactam art for the temporary protection of the carboxylic acid function. These esters are known for their ease of preparation and removal. The carboxylic acid protecting ester groups function to block the carboxylic acid function during the process of this invention to prevent the formation of side products as well as to direct the reaction to the desired carboxylic acid function when two or more are present in the same molecule eg. the compound of Formula (II). Examples of such ester groups are methyl, ethyl, t-butyl, trihaloethyl esters such as the 2,2,2-trichloroethyl ester, allyl, 2-iodoethyl, methoxymethyl, esters formed with tertiary ethinyl carbinols, such as dimethylethinylcarbinol, diethylethinylcarbinol, 1-ethinylcyclopentanol, or 1-ethinylcyclohexanol; the arylalkyl ester forming groups such as benzyl and substituted benzyl, for example, p-nitrobenzyl, p-methoxybenzyl, p-methylbenzyl, diphenylmethyl, and 4-methoxydiphenylmethyl; or the trialkylsilyl esters such as trimethylsilyl, and other conventional carboxy-protecting ester groups.

Examples of substituted methyl groups represented by Y in Formula (I), when Y is a group $-CH(R_3)COOR_4$, are carboxymethyl, methoxycarbonylmethyl, ethoxycarbonylmethyl, benzyloxycarbonylmethyl, dicarboxymethyl, diethoxycarbonylmethyl, methylsulfinylcarboxymethyl, benzoylcarboxymethyl, p-nitrobenzoylcarboxymethyl, cyanocarboxymethyl, acetylcarboxymethyl, acetylethoxycarbonylmethyl, phenylsulfonylcarboxymethyl, methyl-

sulfinylcarboxymethyl, phenylsulfinylcarboxymethyl, 1-carboxyethyl, 1-ethoxycarbonylethyl, 3-carboxypropenyl, or 3-carboxypropynyl. Examples of substituted methyl groups represented by Y, when Y is a substituted sulfomethyl group represented by the radical $-CH(R_3)SO_3H$, are sulfométhyl, 1-sulfoethyl, 1-sulfopropyl, 1-sulfopropenyl, 1-sulfopropynyl, carboxysulfomethyl, ethoxycarbonylsulfomethyl, or acetylsulfomethyl. Representative of the substituted methyl groups when Y in Formula (I) is a substituted vinyl group are 2-phenylvinyl (styryl), 1-carboxyvinyl, 2-carboxyvinyl, 1-ethoxycarbonylvinyl, 2,2-diethoxycarbonylvinyl, 1-ethoxycarbonyl-2,2-diphenylvinyl, 1,2-dimethyl-2-phenylvinyl, or 2-phenyl-2-ethoxycarbonylvinyl.

The compounds represented by Formula (I) in which a free amino or free carboxy group is present can be in salt form. For instance when R represents amino, the acid addition salts are formed with suitable acids such as hydrochloric acid, sulfuric acid, hydrobromic acid, trifluoroacetic acid, or formic acid. Likewise, any free carboxy group can be in the salt form such as the sodium salt, the potassium salt, the ammonium salt, and salts formed with basic amines, for example, the lower alkyl primary, secondary, and tertiary amines such as diethylamine, ethylamine, methylamine, dicyclohexylamine, hexylamine, ethanolamine, or diethanolamine. Such salts may be useful in the isolation and purification of the compounds of the invention.

Compounds represented by Formula (I) in which R is a free amino group are more stable in salt form

and accordingly are stored for future use in the salt form. The hydrochloride salt is a preferred salt form of the 3-aminoazetidinones.

Examples of compounds represented by Formula (I) in which R is an amino group include the following:

N-(ethoxycarbonylmethyl)-3-amino-2-azetidinone,

N-[(diethoxycarbonyl)methyl]-3-amino-2-azetidinone,

N-[(diethoxycarbonyl)methyl]-3-amino-4-methyl-2-azetidinone,

N-(carboxymethyl)-3-aminoazetidinone,

N-(diethoxycarbonylmethyl)-3-amino-4-carboxy-2-azetidinone,

N-(diethoxycarbonylmethyl)-3-amino-4-hydroxymethyl-2-azetidinone,

N-(2-methyl-1-ethoxycarbonylpropen-1-yl)-3-amino-2-azetidinone,

N-(1-ethoxycarbonylpropene-2-yl)-3-amino-2-azetidinone,

N-(sulfomethyl)-3-amino-2-azetidinone,

N-(cyanoethoxycarbonylmethyl)-3-amino-4-ethyl-2-azetidinone,

or an acid addition salts thereof.

Examples of compounds of Formula (I) in which R is a protected-amino group are the following:

N-(diethoxycarbonylmethyl)-3-phthalimido-2-zetidinone,

N-(dibenzyloxycarbonylmethyl)-3-phthalimido-2-azetidinone,

N-[di(4-nitrobenzyloxycarbonyl)methyl]-3-benzyloxycarbonylamino-2-azetidinone,

N-[di(diphenylmethoxycarbonyl)methyl]-3-adamantyloxycarbonylamino-4-methyl-2-azetidinone,

N-(diethoxycarbonylmethyl)-3-(4,5-diphenyl-4-oxazolin-2-one-3-yl)-2-azetidinone,

N-[di(4-nitrobenzyloxycarbonyl)methyl]-3-(4,5-diphenyl-4-oxazolin-2-one-3-yl)-2-azetidinone,

N-(dibenzyloxycarbonylmethyl)-3-(4,5-diphenyl-4-oxazolin-2-one-3-yl)-4-diphenylmethoxycarbonyl-2-azetidinone,

N-(diphenylmethoxycarbonylmethyl)-3-(4,5-diphenyl-4-oxazolin-2-one-3-yl)-2-azetidinone,

N-(sulfomethyl)-3-(4,5-diphenyl-4-oxazolin-2-one-3-yl)-4-hydroxyethyl-2-azetidinone, and

N-(1-ethoxycarbonyl-2-methylpropene-1-yl) 3-(4,5-diphenyl-4-oxazolin-2-one-3-yl)-3-chloromethyl-2-azetidinone.

Examples of compounds of Formula (I) in which R is an acylamino group include the following:

N-(carboxymethyl)-3-phenylacetamido-2-azetidinone,

N-(carboxymethyl)-3-phenoxyacetamido-2-azetidinone,

N-(diethoxycarbonylmethyl)-3-benzamido-2-azetidinone

N-(sulfomethyl)-3-(4-methylbenzamido)-4-methyl-2-azetidinone,

N-(sulfocarboxymethyl)-3-(2-thienylacetamido)-2-azetidinone,

N-(1-methyl-2-carboxyvinyl)-3-(α-carboxyphenylacetamido)-2-azetidinone,

N-(dibenzyloxycarbonylmethyl)-3-(α-sulfophenylacetamido)-2-azetidinone,

N-(t-butyloxycarbonylmethyl)-3-formamido-4-carboxy-2-azetidinone,

N-(1-ethoxycarbonyl-2-methylpropen-1-yl)-3-methoxy-3-(2-thienylacetamido)-4-methyl-2-azetidinone,

N-(carboxymethyl)-3-(α-hydroxyphenylacetamido)-2-azetidinone,

N-(carboxymethyl)-3-(α-aminophenylacetamido)-2-azetidinone,

N-(sulfomethyl)-3-[2-(2-aminothiazol-4-yl)-2-methoxyiminoacetamido]-4-methyl-2-azetidinone,

N-sulfomethyl-3-[2-(4-hydroxyphenyl)-2-(4-ethylpiperazine-2,3-dione-1-yl)carbonylamino]-2-azetidinone,

N-(carboxymethyl)-3-[2-(phenyl)-2-(2,4-dihydroxybenzamido]-2-azetidinone,

N-(sulfomethyl)-3-[2-(2-furyl)-2-methoxyiminoacetamido]-2-azetidinone,

N-(sulfomethyl)-3-[2-(4-hydroxyphenyl)-2-(3-methylcarbamoyl-3-methylureido)acetamido]-2-azetidinone,

N-(1-methyl-2,2-diethoxycarbonylvinyl)-3-cyanoacetamido-4-carboxy-2-azetidinone,

N-(1-sulfoprop-1-yl)-3-(3,4-dichlorophenylmercaptoacetamido)-2-azetidinone,

N-(1-carboxyethyl)-3-acetamido-2-azetidinone,

N-(3-ethoxycarbonylbutine-3-yl)-3-phenylacetamido-2-azetidinone,

N-(carboxymethyl)-3-[2-(2-aminothiazol-4-yl)-2-methoxyiminoacetamido]-2-azetidinone,

N-(diethoxycarbonylmethyl)-3-[2-(2-aminothiazol-4-yl)-2-methoxyiminoacetamido]-3-methyl-2-azetidinone,

N-[di(4-nitrobenzyloxycarbonyl)methyl]-3-[2-(2-tritylaminothiazol-4-yl)]-3-(4-nitrobenzyloxycarbonyl)-2-azetidinone, and

N-(carboxymethyl)-3-[2-(2-aminothiazol-4-yl)-2-(2-carboxyprop-2-yl)oxyiminoacetamido]-2-azetidinone.

Also, in accordance with the invention, there is provided a process for preparing a compound of Formula (I), as defined earlier, which comprises ring-closure of a compound of Formula (II)

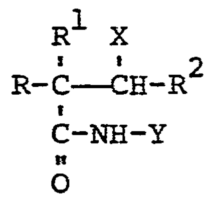

(II)

in which R, $R^1$, $R^2$ and Y are as defined as above and X is hydroxy or halo. In particular a β-hydroxy or β-halocarboxylic acid represented by Formula (III):

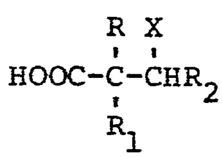

(III)

in which X is hydroxy or halo, is treated with an amine, $H_2N$-Y to produce the substituted amide represented by Formula (II):

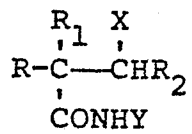

(II)

The substituted amide then is cyclized to the β-lactam, a 2-azetidinone represented by Formula (I):

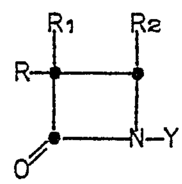

(I)

In the above formulae R, $R_1$, $R_2$, X, and Y have the same meanings as defined earlier for Formula (I).

The substituted amide (II) is prepared by reacting the amine $NH_2Y$ with an activated derivative of the carboxylic acid such as an acid halide, acid azide, acid anhydride, or a so-called active ester. Acid halides, such as the acid chloride or acid bromide, are reacted with the amine at ordinary temperatures in an inert solvent in the presence of an acid binding agent, e.g. triethylamine. When R in the above formula is a protected-amino, acylamino, or diacylamino group, an active ester preferably is used to form the substituted amide. Active esters such as those formed with N-hydroxyimides e.g. N-hydroxysuccinimide and N-hydroxyphthalimide, and with N-hydroxybenzotriazole (HBT) may be used in the process. A preferred active ester is the HBT ester. Active esters prepared with the acid

and methyl chloroformate or isobutyl chloroformate may also be used.

In preparing the substituted amides of Formula (II) in which R is a protected-amino, acylamino or diacylamino group, the protected amino acid, HBT, the amine $NH_2Y$, and a carbodiimide are reacted in an unreactive solvent such as methylene chloride, dimethylformamide, dimethylacetamide or acetonitrile. The protected amino acid condense with the HBT, in the presence of the carbodiimide, to form the active ester which then reacts with the $H_2NY$ amine to form the substituted amide. Carbodiimides such as the water insoluble dicyclohexylcarbodiimide can be used, or a water soluble carbodiimide such as 1-cyclohexyl-3-(2-morpholinoethyl)carbodiimide metho-p-toluene sulfonate may be used.

The primary amines, $H_2NY$, used to prepare the substituted amides II are represented by Formulae (a), (b), and (c):

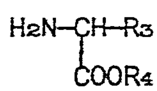 , 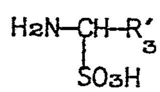 , 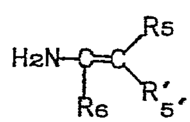

(a) (b) (c)

Examples of the amines which may be employed in the process include ethyl glycinate, methyl glycinate, ethyl cyanoglycinate, p-methoxybenzyl glycinate, p-nitrobenzyl glycinate, diphenylmethyl glycinate, diethyl aminomalonate, dimethyl aminomalonate, ethyl-2-amino-3-oxobutyrate, ethyl α-sulfoglycinate, ethyl α-benzoylaminoglycinate, ethyl α-aminopropionate, aminomethanesulfonic acid, ethyl 3-aminocrotonate, and dehydrovaline.

Examples of β-substituted acids which may be used in the process are the amino acids, serine, threonine, β-hydroxyglutamic acid, β-hydroxylysine, β-hydroxyaspartic acid, in which the amino group is suitably protected, and the β-substituted acids; malic acid, chloro and bromo succinic acid, chloropivalic acid, bromopivalic acid, α-methoxy-β-hydroxybutyric acid, tartaric acid, α,β-dihydroxyhexanoic acid, or α-(2-hydroxyethyl)-β-hydroxybutyric acid.

The cyclization to the azetidinone of the substituted amide of Formula (II) in which X is hydroxy and Y is a sulfomethyl, esterified carboxymethyl, or substituted vinyl group and which is formed from amines a, b, or c, is carried out in an inert anhydrous solvent with an organo phosphorous compound selected from triphenylphosphine, triphenylphosphite, diphenyl phenylphosphonate, and phenyl diphenylphosphinoate, and a dialkylazodicarboxylate. The cyclization occurs readily under mild conditions, for example, at a temperature between about 15°C. and about 40°C. and conveniently at or about ambient room temperature. The organo phosphorous compound and the dialkylazodicarboxylate are each used in about 100 mole percent to about 150 mole percent relative to the substituted amide II. Solvents which may be used are desirably the lower boiling aprotic organic solvents in which the starting material and the reactants are soluble. Tetrahydrofuran (THF) is

a preferred solvent while other common solvents such as acetonitrile, diethyl ether, ethyl acetate, methylene chloride, dimethylformamide, and dimethylacetamide may be used as well as mixtures of such organic solvents. THF is a preferred solvent because it is easy to remove from the reaction mixture. Preferred dialkylazodicarboxylates are diethylazodicarboxylate and diisopropylazodicarboxylate.

A preferred organo phosphorus compound is triphenylphosphine. The cyclization is carried out by adding the dialkylazodicarboxylate to a solution of the substituted amide II and the organo phosphorus with agitation. The cyclization usually is complete in about one hour or less when the process is conducted on a small scale with the preferred triphenylphosphine. For large scale manufacturing the reaction should be completed in somewhat longer times.

In an example of the process N-benzyloxycarbonyl L-serine is coupled with diethylaminomalonate to form the dipeptide (substituted amide II in which R is protected-amino, X = OH, $R_1$=$R_2$=H, and Y is $-CH(COOC_2H_5)$. The dipeptide is reacted with triphenylphosphine-diethylazodicarboxylate to form, as shown in the following reaction scheme, the azetidinone N-(diethoxycarbonylmethyl)-3-benzyloxycarbonylamino-2-azetidinone and a minor amount of the aziridine side product.

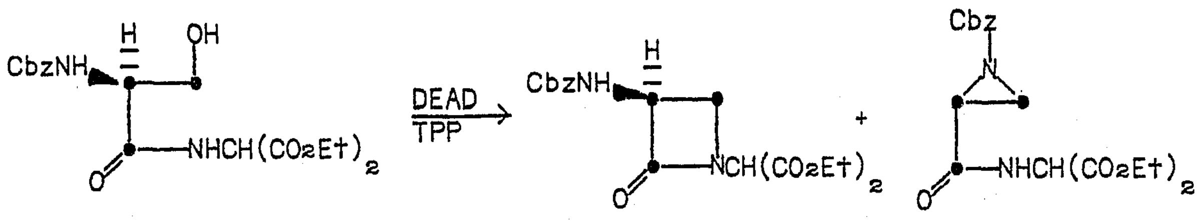


In carrying out the process, any free carboxy groups, other than that required to form the desired amide with $H_2NY$, are protected during the process with a conventional carboxy-protecting ester group. Likewise any free amino groups, such as when R is amino, are protected with an amino-protecting group during the process.

When the process is carried out with a β-halo substituted amide (Formula II, X = halo), the cyclization to the β-lactam is effected with a strong base such as a lithium dialkylamide eg. lithium di-tert-butylamide, or lithium diisopropylamide and like lithium amides or lithium hexamethyldisilazide. The cyclization is carried out under anhydrous conditions in an inert aprotic organic solvent such as THF, dimethylformamide or dimethylacetamide. Mixtures of such solvents also may be used to form the reaction medium.

A preferred base is a lithium dialkylamide and, in particular, lithium diisopropylamide. With the preferred base the reaction is carried out initially at a temperature between about -80°C. and about -25°C. at which temperature the anionic form of the substituted amide is generated. The reaction then is allowed to warm to a temperature of between about 10°C. and

about 25°C. during which time β-lactam ring formation occurs. Approximately 200 mole percent of a preferred base relative to the substituted amide is used for best yields of the β-lactam compound. When used at 100 mole percent the desired β-lactam product is obtained in only minor amounts, and, in the instance in which Y is a sulfo-substituted methyl group or an esterified carboxy-substituted methyl group (formed with amines a and b), the substituted 2-pyrrolidone is the major product.

According to the process of this invention a selective cyclization to the 4-membered β-lactam ring is observed. N-alkylation of the amide nitrogen of the β-substituted amide (Formula (II) occurs rather than C-alkylation of the α'-carbon bearing the electron withdrawing group(s). C-Alkylation of the active methylene group would be expected since the acidity of the amide N-H bond and the α'-CH bond should differ only slightly. For example, cyclization via C-alkylation of Cbz-protected serylaminomalonate represented by the formula:

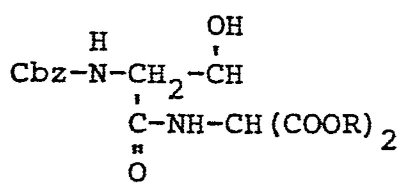

would be expected to yield the 5-membered pyrrolidone represented by the following formula:

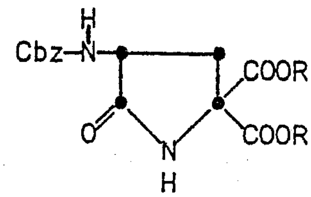

It has been found, however, that the greater the acidity of the α'-CH proton the more selective the amide N-H alkylation becomes and the more the 4-membered β-lactam product predominates.

The preferred process of this invention for the preparation of the β-lactam compounds of Formula (I) shown below

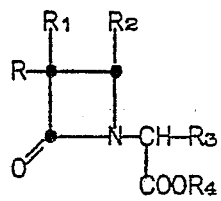

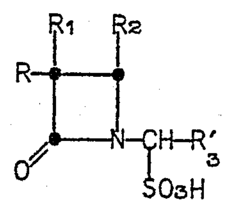

is the cyclization of a β-hydroxy substituted amide II (X=OH) with triphenylphosphine and a dialkylazodicarboxylate.

A further preferred embodiment of the process for the preparation of a compound of the Formula (I) in which Y is a group of the formula

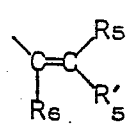

comprises the cyclization of a β-halo substituted amide (II).

A further preferred process of this invention comprises the use of a β-hydroxy substituted amide represented by the Formula

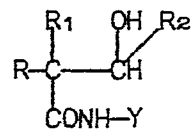

in which R is protected-amino, acylamino, or diacylamino, and the cyclization thereof to a 3-protected-amino-, acylamino-, or diacylamino-2-azetidinone. In particular, R is a protected-amino group in the process and Y is a group represented by the formulae.

$$\begin{array}{l} -CH-R_3 \\ \;\;COOR_4 \end{array} \qquad \begin{array}{l} -CH-R_3' \\ \;\;SO_3H \end{array}$$

Preferred protected-amino groups in this embodiment of the invention are the carbamate protecting groups as defined earlier and in particular the benzyloxycarbonylamino and p-nitrobenzyloxycarbonylamino protected-amino groups. A particularly preferred protected-amino group is the 4,5-diphenyl-4-oxazolin-2-one group ("Ox" protecting group) represented by the following formula:

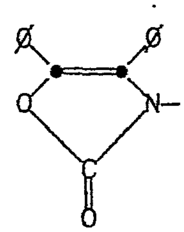

In a further preferred embodiment, R is a preferred amino-protected amino group and $R_3$ is hydrogen, $C_1$-$C_4$ alkoxycarbonyl, or protected-carboxy, and $R_4$ is $C_1$-$C_4$ alkyl or a carboxy protecting group. In an example of the preferred embodiment N-Cbz-L-serine is converted to the active HBT ester with HBT and dicyclohexylcarbodiimide and the ester allowed to react with diethyl aminomalonate to form the N-Cbz-L-serine-aminomalonate dipeptide represented by the formula,

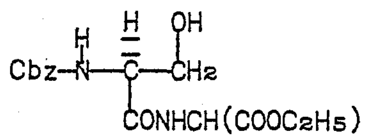 .

The dipeptide is reacted with 100 mole percent of TPP and about 100 mole percent of diethylazodicarboxylate to provide the β-lactam product, N-[(diethoxycarbonyl)-methyl]-3-benzyloxycarbonylamino-2-azetidinone and the aziridine side product as shown in the following reaction scheme,

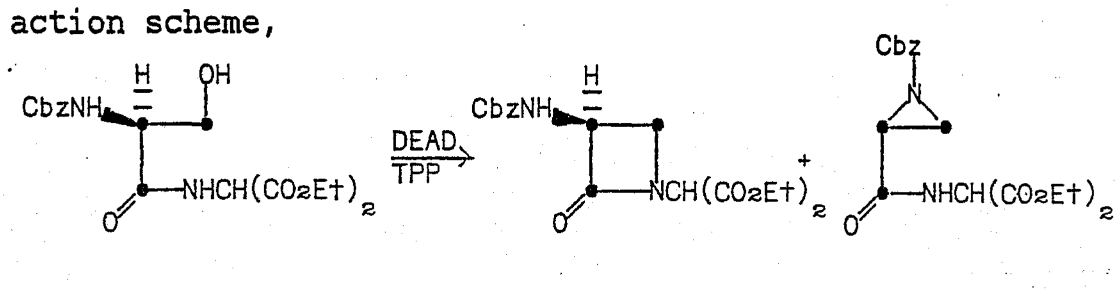


in which Cbz = benzyloxycarbonyl; DEAD = diethylazodicarboxylate, and TPP = triphenylphosphine. The mixture is separated by chromatography to provide the purified β-lactam compound.

In another preferred embodiment of the invention, L-serine is reacted with 1,2-diphenylvinylene carbonate to provide the Ox protected-L-serine and the latter is converted to the HBT ester and coupled with diethyl aminomalonate to form the dipeptide (Formula (II), R = protected-amino, X = OH, $R_1 = R_2 =$ H, and Y = diethoxycarbonylmethyl). The Ox protected dipeptide is reacted with TPP and diisopropylazodicarboxylate (DIAD) to provide the 3-Ox-protected-amino β-lactam as shown in the following reaction scheme:

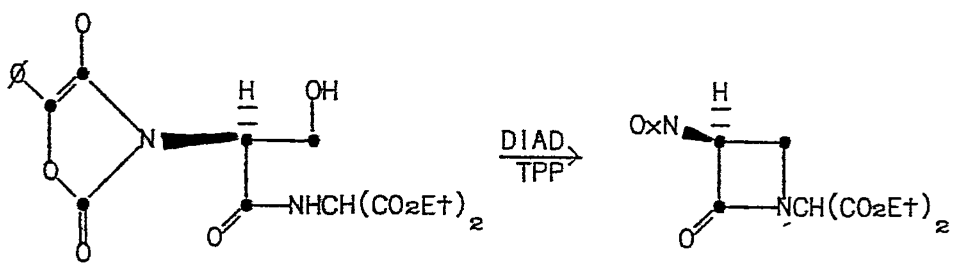


In a further preferred embodiment of the invention L-threonine is converted to the Ox-protected derivative, coupled with diethyl aminomalonate and, the dipeptide obtained is converted with TPP and diisopropylazodicarboxylate to the Ox-protected 3-amino-2-azetidinone as shown in the following reaction scheme:

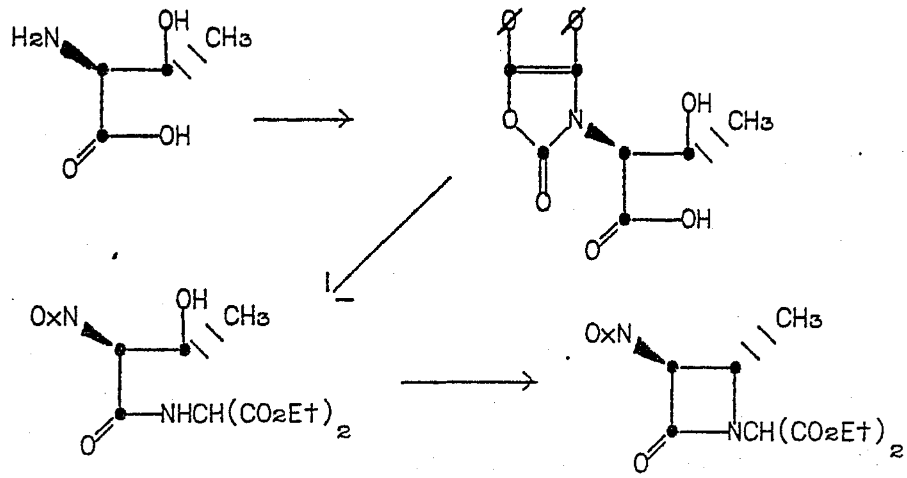

The amino-protecting group of the 3-protected amino-2-azetidinones is removed to provide the 3-amino-2-azetidinones. For example in the above preferred embodiments the Cbz protecting group is removed by hydrogenolysis using palladium on carbon catalyst. The Ox protecting group is removed likewise as shown below.

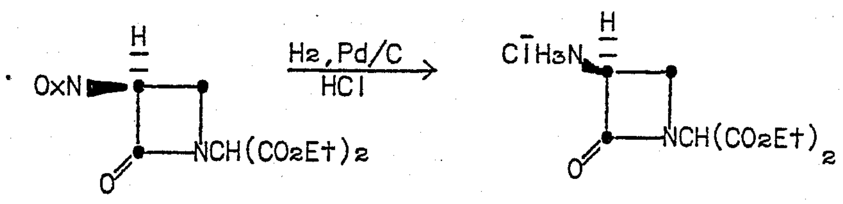


The 3-amino-2-azetidinones (Formula (I), R = $NH_2$), can be acylated with an active derivative of the desired carboxylic acid to provide a compound of Formula (I) in which R is an acylamino group.

Alternatively, the 3-acylamino-2-azetidinones (Formula (I)) can be obtained in the process of the invention as described earlier. However, in the process, an acylamino-substituted amide (Formula (II), R = acylamino) undergoes cyclization to form the oxazoline side product as well as the β-lactam formation. Accordingly, the 3-acylamino-2-azetidinones preferably are prepared via acylation of a 3-amino-2-azetidinone obtained with a 3-protected-amino-azetidinone as described above.

Preferred 3-acylaminoazetidinones of the invention are represented by Formula (I) in which R is amino, protected-amino or an acylamino group. Further preferred 3-acylamino-2-azetidinones are represented by the formula,

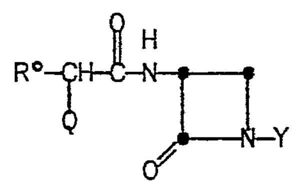

in which Y is $-CH_2COOH$ or $-CH_2SO_3H$.

Examples of such preferred compounds are N-carboxymethyl-3-phenylglycylamino-2-azetidinone, N-sulfomethyl-3-mandeloylamino-2-azetidinone, N-carboxymethyl-3-(2-thienylacetylamino)-2-azetidinone, N-carboxymethyl-3-mandeloylamino-2-azetidinone, N-sulfomethyl-3-mandeloylamino-2-azetidinone, N-carboxymethyl-3-(α-carboxyphenylacetylamino)-2-azetidinone, N-carboxymethyl-3-[α-carboxy-(4-hydroxyphenyl)acetyl-

amino]-2-azetidinone, N-sulfomethyl-3-(α-sulfophenyl-acetylamino)-2-azetidinone and N-carboxymethyl-3-(α-sulfophenylacetylamino)-2-azetidinone.

A further preferred group of compounds of the invention are represented by the following formula:

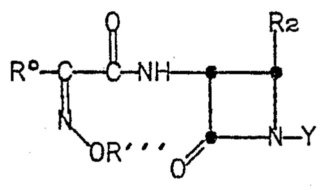

in which Y is a carboxymethyl or sulfomethyl group in which $R_3$ and $R_4$, independently, are hydrogen or methyl, and R° and R''' have the same meanings as defined for Formula (I). Preferably R° is 2-furyl, 2-thienyl, or 2-aminothiazol-4-yl, and R''' is methyl, carboxymethyl, or 2-carboxyprop-2-yl. Examples of the preferred compounds are N-sulfomethyl-3-[2-(2-aminothiazol-4-yl)-2-methoxyiminoacetamido]-4-methyl-2-azetidinone, N-sulfomethyl-3-[2-(2-aminothiazol-4-yl)-2-(2-carboxyprop-2-yl)oxyiminoacetamido]-4-methyl-2-azetidinone, N-carboxymethyl-3-[2-(2-aminothiazol-4-yl)-2-methoxyiminoacetamido]-2-azetidinone, N-carboxymethyl-3-[2-(2-furyl)-2-methoxyiminoacetamido]-4-methyl-2-azetidinone, N-carboxymethyl-3-[2-(2-thienyl)-2-(2-carboxyprop-2-yl)oxyiminoacetamido]-2-azetidinone, or a sodium or potassium salt thereof.

The β-lactam compounds represented by the Formula (I) are intermediates useful in the preparation

of antibiotics and β-lactamase inhibitors. A number of preparative routes have been devised for the penem, penam, oxapenem, oxacephalosporins, clavulanic acid, monobactam, and thienomycin structures, virtually all of which employ a substituted 2-azetidinone. The compounds (Formula (I)) of this invention can be used in such preparative routes or can be converted by methods known in the art to structures which in turn can be used as intermediates. For example, in mentioning only a few of such routes, compounds of Formula (I) in which $R_1$ is hydrogen, $R_2$ is carboxy, and Y is a carboxy protected carboxymethyl group can be converted to the 2-substituted penams via the corresponding 4-methylthio-2-azetidinones as described by Foxton, M.W. et al., "Recent Advances in the Chemistry of β-lactam Antibiotics", G.I. Gregory (ed), 1981 p. 281; and Tetrahedron Letters, 1981, 22, 2479.

Likewise, the 2-substituted penems described by Woodward, R.B. et al., J. Amer. Chem. Soc., 1978, 100, 8214, can be prepared via the 4-acylthio-2-azetidinones obtained by known methods with compounds of the invention.

Compounds of the invention in which R is hydroxy-substituted alkyl (eg. ethyl) and Y is a carboxy substituted vinyl group can be used in the preparation of thienamycin described by Karady, S. et al., J. Amer. Chem. Soc., 1981, 103, 6765, or the method described by Schmitt, S.M. et al., J. Org. Chem. 1980, 100, 313.

The compounds in which Y is a substituted vinyl group can be converted by known oxidative cleavage methods, eg. with ozone, to the N-unsubstituted azetidinone and the latter N-alkylated to nocardian type antibiotics or to the antibiotic substance "PS-5" by using the procedures described by Kametani, T. et al., Heterocyclics, 1982, 19, 1023, and Kametani, T., Heterocyclics 1982, 17, 463, 479 for carbapenem synthesis via N-substituted methylazetidin-2-ones.

Clavulanic acids and analogs thereof may be obtained with compounds of the invention by the methods described by Bently, P.H. et al., in "Recent Advances in the Chemistry of β-Lactam Antibiotics", 1981, p. 175, Tetrahedron Letters 1979, 1889 and, also Parker, R.M. et al., J. Chem. Soc. Chem. Commum., 1977, 905, 906. 1-Oxapenams (oxabisnorpenicillins) may be made with compounds of the invention by conversion by known methods to the starting materials employed by Cama, L.D. et al., Tetrahedron Letters, 44, 4233 in preparing the 1-oxapenams.

Certain of the compounds of the invention are useful intermediates for preparing the 3-acylamino-2-azetidinones described above. For example, when R in Formula (I) is the 3-protected-amino or the 3-amino group, the compounds represented are useful in the preparation of the 3-acylamino-substituted azetidinone antibacterials.

For example, the protecting group of the amino-protected β-lactam is removed to provide the 3-amino-β-lactam. The latter nucleus compound is then acylated by employing acylation methods commonly used to acylate other β-lactam nucleus compounds such as 6-APA and 7-ACA. Such methods include the use of an active derivative of the carboxylic acid acylating moiety such as the acid halides, and active esters.

Active esters such as those prepared with the acid and methyl chloroformate or isobutyl chloroformate, or with the N-hydroxy heterocyclics such as N-hydroxybenzotriazole, and N-hydroxysuccinimide can be used. During the acylation of a 3-amino-2-azetidinone compound any free carboxy or other amino groups in the molecule are protected by conventional means.

For example, N-(diphenylmethoxycarbonylmethyl)-3-amino-2-azetidinone is reacted in THF-water with phenoxyacetyl chloride in the presence of triethylamine to provide N-(diphenylmethoxycarbonylmethyl)-3-phenoxyacetamido-2-azetidinone. Treatment of the product with trifluoroacetic acid in the presence of anisole removes the diphenylmethyl ester group to provide the N-(carboxymethyl)azetidinone.

Acylation of N-[(dimethoxycarbonyl)methyl]-3-amino-4-benzyloxycarbonyl-2-azetidinone with N-t-Boc protected-D-phenylglycine in the form of the active ester formed with isobutyl chloroformate provides, after deblocking the α-amino group and the $C_4$ carboxy group, N-[(dimethoxycarbonyl)methyl]-3-D-phenylglycylamino-4-carboxy-2-azetidinone. Saponification forms the N-(carboxymethyl) compound.

The compounds of the invention in which Y is a dialkoxycarbonylmethyl group are converted to compounds in which Y is carboxymethyl by saponification under mild conditions. Upon de-esterification to the N-dicarboxymethyl compound, decarboxylation results forming the N-carboxymethyl substituted 2-azetidinone.

The compounds of Formula (I) in which $R_2$ is sulfhydryl, $C_1$-$C_4$ alkylthio, $C_2$-$C_4$ alkanoylthio or

halo, are prepared after cyclization with compounds of the Formula (I) in which $R_2$ is a group convertible, by known methods, to halo, for example the carboxy group. The halo-substituted compound then can be converted to the -SH, -S-alkyl, or -S-alkanoyl group.

To further illustrate the invention, the following non-limiting examples are provided. In the examples, melting points were taken on a Thomas-Hoover melting point apparatus and are uncorrected. Infrared spectra were recorded on a Perkin-Elmer 727b spectrometer. NMR spectra were obtained in chloroform-d with tetramethylsilane as a reference, unless otherwise stated, on a Varian EM390, XL-100 or Nicolet NB300 Spectrometer. Mass spectra were recorded on an AEI Scientific Apparatus 902 or Dupont DP102 spectrometer. High pressure LC was performed using a Beckman/Altex Model 332 chromatograph. Medium pressure chromatography was executed with the Michel-Miller system packed with Silica Gel 60 (40-63 μ).

The following abbreviations are used in the examples: DEAD = diethylazodicarboxylate; DIAD = diisopropylazodicarboxylate; THF = tetrahydrofuran; DMF = dimethylformamide; Cbz = carbobenzoxy (benzyloxycarbonyl); IPA = isopropyl alcohol; TPP = triphenylphosphine; LDA = lithium diisopropylamide; DCHA = dicyclohexylamino; Ox refers to the following amino-protecting group:

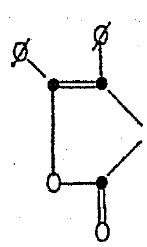

General procedures for the preparation of dipeptides.

Method A. The amino-protected amino acid, 2 eq. of N-hydroxybenzotriazole (HBT), ca. 1.2 eq. of the amine hydrochloride and 1 eq. of triethylamine are dissolved in methylene chloride or DMF. Dicyclohexylcarbodiimide DCC (ca. 1.2 eq) is added and the reaction mixture stirred for about 4 hours to about 20 hours. The resulting insoluble dicyclohexylurea is separated by filtration and the filtrate diluted with diethylether or with ethyl acetate. The solution is washed with 10% sodium carbonate, 0.1N hydrochloric acid, water (only when DMF was the solvent), brine, and then dried over magnesium sulfate. The purified product is isolated after evaporation, chromatography (where necessary) and/or recrystallization.

Method B. Alternatively, the water and methylene chloride soluble carbodiimide, 1-cyclohexyl-3-(2-morpholinoethyl)carbodiimide metho-p-toluene-sulfonate (Aldrich) is used instead of DCC. Since the urea produced is water soluble, no chromatographic purification is necessary.

Example 1

N-(Methoxycarbonylmethyl)-3-phthalimido-2-azetidinone

Methyl (L)-$N^{\alpha}$-phthaloylserylglycinate was prepared from (L)-N-phthaloylserine and methyl glycine by method A in 50% yield and isolated as a foamy residue

which was homogenous by TLC [75/25 ethyl acetate and hexanes]. NMR (δ): 3-3.4 (1H, br), 3.73 (3H, s), 3.84-4.6 (3H, m), 4.9-5.06 (1H, m), 7.6-8.0 (5H, m).

The phthaloylserylglycinate ester (0.66 g, 2.16 mmoles) was treated with 0.57 g (2.18 mmoles) of TPP and .34 mL (2.16 mmoles) of DEAD in 30 mL of THF for 3 hours. NMR of the crude mixture showed starting material, the title compound and the dehydropeptide in a ratio of 2:5:3. Chromatography [silica, 80/20 hexanes and ethyl acetate] gave two fractions containing the products. From fraction 2 were crystallized 75.4 mg (.26 mmoles) of the title compound. M.P. 178-180°C. IR ($CHCl_3$): 1785 (sh), 1770, 1750, 1720 $cm^{-1}$. MS: m/e 290 (M+1). Homogenous by HPLC [10 μ silica, 250 x 3.2 mm, .4% IPA in methylene chloride, 5 mL/min, .25 cm/min, Rt 9.6 min.] NMR (δ): 3.83 (3H, s), 3.86-3.98 (2H, m), 3.99-4.5 (2H, q, J = 16.5 Hz), 5.53-5.6 (1H, dd), 7.6-7.9 (4H, m).

Analysis calculated for $C_{14}H_{12}N_2O_5$:

C, 58.13; H, 4.15; N, 9.69

Found: C, 58.31; H, 4.05; N, 9.67.

Fraction 3 contained a mixture of the desired title compound and the dehydropeptide and was rechromatographed on a TLC column [80/20/.8 methylene chloride, hexanes, IPA]. 63 mg (.22 mmoles) of the β-lactam title compound were isolated, but the dehydropeptide decomposed on the column. The total yield of the title compound was 22%.

Example 2

N-[(Diethoxycarbonyl)methyl]-3-phthalimido-2-azetidinone

Diethyl (L)-N-phthaloylserylaminomalonate was prepared (method A) in 64% yield. M.P. 103-105°C (ethyl acetate and hexanes). NMR (δ): 1.17-1.37 (6H, dt), 3.1-3.3 (1H, m), 4.0-4.5 (6H, m), 4.93-5.07 (1H, m), 5.12-5.2 (1H, d), 7.6-8 (5H, m).

Analysis calculated for $C_{18}H_{20}N_2O_8$:

C, 55.1; H, 5.1; N, 7.12.

Found: C, 54.97; H, 5.22; N, 7.12.

The seryl-aminomalonate dipeptide (0.5 g, 1.28 mmoles) was treated with 0.34 g (1.28 mmoles) of TPP and 0.20 mL (1.28 mmoles) of DEAD in 40 mL of THF for 1.5 hours. The reaction was followed by HPLC and TLC, and shown to be complete in 15 minutes. The title compound and the corresponding dehydropeptide co-eluted on silica gel chromatography [9:1 methylene chloride and ether]. The desired β-lactam (title compound) crystallized selectively from ethyl acetate and hexanes to give 0.2473 g (.67 mmoles, 52%). M.P. 149-150°C. IR (KBr): 1780, 1760, 1730 $cm^{-1}$. MS: (CI with methane): m/e 375 (M+1). NMR (δ): 1.19-1.39 (6H, dt) 4.03-4.47 (6H, m), 5.37 (1H, s), 5.47-5.57 (1H, m), 7.6-7.9 (4H, m). $^{13}C$ NMR: 15.81, 48.2, 55.8, 58.52, 64.3, 125.3, 133.4, 136.0, 165.9, 166.3, 166.9, 168.2. Single peak by HPLC [10 μ silica, 250 x 3.2 mm, 5 mL/min, .25 cm/min, Rt 6.48 min., 898:100:2 methylene chloride, hexanes, IPA].

Analysis calculated for $C_{18}H_{18}N_2O_7$:
C, 57.75; H, 4.81; N, 7.49.
Found: C, 57.44; H, 5.04; N, 7.38.

The supernatant from the crystallization was enriched in the dehydropeptide side product but it could not be crystallized selectively. HPLC conditions: [the same as for title compound, Rt = 6 minutes] NMR (δ): 1.19-1.39 (6H, dt), 4.0-4.4 (4H, m), 5.17-5.27 (1H, d), 5.93 (1H, m), 6.3 (1H, m), 7.0-7.2 (1H, br d), 7.6-7.9 (4H, m).

## Example 3

N-[(Diethoxycarbonyl)methyl]-3-benzyloxycarbonylamino-2-azetidinone

Diethyl (L)-$N^{\alpha}$-Cbz serylaminomalonate was prepared (method B) from (L)-N-Cbz-serine and diethyl aminomalonate in 75% yield. M.P. 85-88°C. (dec.). NMR (δ): 1.1-1.3 (6H, m), 3.6-4.5 (8H, m), 5.06 (2H, s), 5.13-5.23 (1H, d), 7.3 (5H, s and 1H, br.).

Analysis calculated for $C_{18}H_{24}N_2O_8$:
C, 54.54; H, 6.06; N, 7.07.
Found: C, 54.40; H, 6.04; N, 7.15.

The Cbz protected serylaminomalonate (0.5 g, 1.2 mmoles) and 0.33 g (1.2 mmoles) of TPP in 30 mL of THF were treated with 0.2 mL (1.29 mmoles) of DEAD for 1 hour at R.T. The mixture was chromatographed as above, yielding 0.29 g (63% mass recovery) of the mixture of the title compound and 1-Cbz-2-carboxyaziridine diethoxycarbonylmethylamide. 105 mg of the mixture were placed on preparative TLC plates (silica

gel, 2 mm, ether/hexanes, 4 times). The title compound eluted first, and after evaporation of residual solvent, 64.1 mg (.18 mmoles, 15%) were isolated as an oil. IR (neat): 1770, 1750, 1715 $cm^{-1}$. NMR (δ): 1.13-1.33 (6H, m), 3.56-3.7 (2H, m), 4.0-4.4 (4H, m), 4.6-4.8 (1H, m), 5.1 (3H, 2 overlapping singlets), 5.8-6 (1H, m), 7.4 (5H, s). The aziridine side product: 39.5 mg (.1 mmoles, 8.7%) IR (neat): 1760, 1730 $cm^{-1}$. NMR (δ): 1.16-1.33 (6H, t), 2.4-2.6 (2H, dd), 3.06-3.16 (1H, dd), 4.1-4.4 (4H, dq), 4.5-4.8 (1H, b), 5.06-5.1 (1H, d), 5.13 (2H, s), 7.4 (5H, s).

## Example 4

N-[(Diethoxycarbonyl)methyl]-3,3-dimethyl-2-azetidinone

Diethyl N-(β-hydroxypivaloyl)aminomalonate was prepared (method B) from β-hydroxy pivalic acid and diethyl aminomalonate in 88% yield. The compound was isolated as an oil. NMR (δ): 1.13-1.3 (12H, m), 3.5 (2H, br s), 4.16-4.4 (4H, q), 5.1-5.16 (1H, d), 7.6-7.7 (1H, d).

The β-hydroxypivaloylaminomalonate (0.5 g, 1.8 mmoles) and 0.5 g (1.9 mmoles) of TPP were dissolved in 20 ml of THF. To this solution was added dropwise 0.33 mL (1.9 mmoles) of DEAD under a nitrogen blanket. After 1 hour the mixture was concentrated and chromatographed (silica, methylene chloride and hexanes) to yield 0.499 g of an oil. Crystallization from hexanes gave 0.27 g (0.64 mmoles, 35%) of the adduct represented by the formula

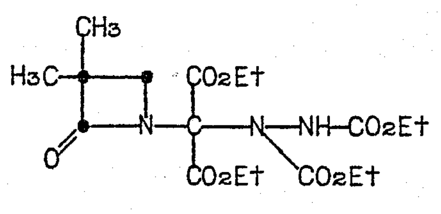

M.P. 68-69°C. IR: 1760, (shoulders at 1740, 1720 $cm^{-1}$). NMR (δ): 1.16-1.4 (18H, m), 3.6 (2H, bs), 4.06-4.43 (8H, overlapping quartets), 7.1-7.3 (1H, bs).

Analysis calculated for $C_{18}H_{24}N_3O_9$:

C, 50.1; H, 6.70; N, 9.7.

Found: C, 50.08; H, 6.70; N, 9.73.

The mother liquor contained exclusively the β-lactam title compound which was isolated as an oil. IR (neat): 1780, 1740 $cm^{-1}$. NMR (δ): 1.16-1.36 (12H, singlet overlapping a triplet), 3.4 (2H, s), 4.13-4.4 (4H, q), 5.16 (1H, s).

Example 5

N-[(Diethoxycarbonyl)methyl]-3,3-dimethyl-2-azetidinone via diethyl N-(β-chloropivaloyl)aminomalonate

Diethyl aminomalonate hydrochloride (1 g, 4.7 mmole) was dissolved in 5 mL of dry pyridine and cooled to 0°C. β-Chloropivaloylchloride (0.72 g, 4.7 mmole) was added dropwise under nitrogen. The mixture was warmed to room temperature and stirred for 2 hours at which time 50 mL of anhydrous ether was added. The precipitate was filtered and the supernatant washed with 0.1 N HCl (4 x 25 mL), 5% $NaHCO_3$

(25 mL), brine (50 mL) and dried over $MgSO_4$. On evaporation, 1.35 g (4.6 mmoles, 98%) of the oily product were isolated. NMR (δ): 1.16-1.33 (12H, m), 3.63 (2H, s), 4.13-4.36 (4H, q), 5.06-5.13 (1H, d), 6.8-6.9 (1H, bd).

A. Cyclization With 100 mole % of Base

The N-(β-chloropivaloyl)aminomalonate ester (0.5 g, 1.7 mmoles) was treated with 75 mg (1.7 mmoles) of pre-washed 50% NaH in 10 mL of DMF/methylene chloride (1:4) for 15 hours under $N_2$. The reaction was quenched in 0.1 N HCl and extracted with ethyl acetate (100 mL). The organic phase was then washed with 0.1 N HCl (25 mL), $H_2O$ (2 x 25 mL), brine (25 mL) and dried over $MgSO_4$. Evaporation, followed by recrystallization from hexanes gave a total of 0.203 g (0.70 mmoles, 46%) of 3,3-dimethyl-5,5-diethyloxycarbonylpyrrolidone-2, IR (KBr): 1720, 1700 $cm^{-1}$; NMR (δ): 1.13-1.3 (12H, m), 2.53 (2H, s), 4.0-4.4 (4H, m), 6.6 (1H, b).

Analysis calculated for $C_{12}H_{19}NO_5$:

C, 56.03; H, 7.39; N, 5.45.

Found: C, 56.18; H, 7.56; N, 5.45.

B. Cyclization With 220 mole % of base

The β-chloropivaloylaminomalonate (155 mg, 0.53 mmole) was dissolved in 6 mL of THF and cooled to -78°C. under $N_2$. LDA (220 mole % from 0.167 mL, 1.2 mmole, of diisopropyl amine and 1 mL of 1.3 M n-BuLi in 3 mL of THF) was added. The light yellow solution of the dianion was allowed to warm slowly to room temperature over 4-5 hours. After continued stirring for 12 hours, the solution was diluted with 75 mL of ether and washed with 25 mL of 0.1 N HCl, 25 mL of 5%

$NaHCO_3$, and 25 mL of brine. Drying over $MgSO_4$, filtering, and evaporation yielded 114.4 mg (0.45 mmole, 84%) of the β-lactam title compound of which was identical to that prepared as described by Example 4.

## Example 6

N-(3-Ethoxycarbonylpropen-2-yl)-3,3-dimethyl-2-azetidinone

Ethyl N-(β-chloropivaloyl)-3-aminocrotonate was prepared in 42% yield from β-chloropivaloyl chloride and ethyl 3-aminocrotonate by the procedure used in Example 5. M.P. 76-77.5°C (after recrystallization from hexanes); IR (KBr): 1705, 1675 $cm^{-1}$; 'H-NMR (δ): 1.06-1.23 (9H, m), 2.3 (3H, s), 3.6 (2H, s), 4.0-4.23 (2H,q) 6.7 (1H, s), 6.8-7.1 (1H, br).

Analysis calculated for $C_{11}H_{18}NO_3Cl$:

C, 53.44; H, 7.29; N, 5.67.

Found: C, 53.10; H, 7.20; N, 5.73.

The β-chloropivaloyl amide cyclization to the title β-lactam compound was accomplished by treatment of 209 mg (0.84 mmole) of the amide with 64.5 mg (1.3 mmole) of NaH in 5 mL of $DMF-CH_2Cl_2$ (1:4) for 3 hours at room temperature. The reaction mixture was poured into 75 mL of ether and washed with two 25 mL portions of water, 25 mL of brine, dried over $MgSO_4$, filtered and evaporated to yield 177 mg (0.84 mmole, 100%) of the β-lactam title compound as an oil. IR (neat): 1770, 1705 $cm^{-1}$; $^1H$-NMR (δ): 1.16-1.33 (9H, m), 2.63 (3H, s), 3.26 (2H, s), 4.03-4.26 (2H, q), 5.2 (1H, s).

## Example 7

N-(2-Methyl-1-ethoxycarbonylpropen-1-yl)-3,3-dimethyl-2-azetidinone

Ethyl N-(β-chloropivaloyl)dehydrovaline was prepared in 20% yield by the procedure used in Examples 5 and 6. M.P. 111-113°C; IR (KBr): 1715, 1640 $cm^{-1}$; 'H-NMR (δ): 1.13-1.36 (3H, t and 6H, s), 1.83 (3H, s), 2.2 (3H, s), 3.67 (2H, s), 4.1-4.3 (2H, q), 6.9-7.2 (1H, br s).

Analysis calculated for $C_{25}H_{20}NO_3Cl$:

C, 55.17; H, 7.66; N, 5.36.

Found: C, 55.02, H, 7.65; N, 5.47.

The dehydrovalineamide was cyclized to the β-lactam title compound in the same manner as described in Example 6. The title compound represented by the formula

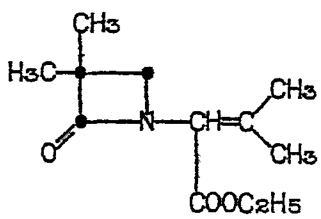

was obtained as an oil in 83% yield. IR (thin film in $CDCl_3$): 1760, 1740, 1720 $cm^{-1}$. $^1$H-NMR (δ): 1.23-1.37 (3H, t and 6H, s); 1.93 (3H, s), 2.23 (3H, s), 3.3 (2H, s), 4.09-4.3 (2H, q).

## Example 8

Diethyl-3-(S)-amino-1-malonyl-2-azetidinone

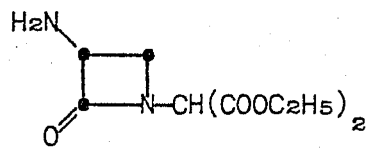

Diethyl $N^{\alpha}$-Ox-L-serylaminomalonate was prepared in 59% yield by method B using N-Ox-L-serine DCHA salt and diethyl aminomalonate. M.P. 104-105°C. (after recrystallization from ethyl acetate-hexanes); NMR (δ): 1.1-1.35 (6H, m), 4.0-4.35 (8H, m), 5.1-5.2 (1H, d), 7.3 (5H, s), 7.5 (5H, s), 8.1-8.15 (1H, brd); $[\alpha]_D^{23}$ = 13.4 (d=0.73, $CH_3OH$).

Analysis calculated for $C_{25}H_{26}N_2O_8$:

C, 62.24; H, 5.39; N, 5.81.

Found: C, 61.89; H, 5.48; N, 5.87.

To 25 mL of dry THF was added 1.1 g (2.5 mmole) of the Ox protected seryl amide and 0.78 g (3 mmole) of TPP. While stirring at room temperature, a solution of 0.5 mL (2.5 mmole) of DIAD in 25 mL of THF was added dropwise over 1 hour. Immediately after completion of the addition, the colorless solution was concentrated and chromatographed (silica gel, $CH_2Cl_2$) to provide 1.1 g (2.45 mmole, 96%) of the desired β-lactam title compound. M.P. 94-95°C (after recrystallizing from ethyl acetate-hexanes); $[\alpha]_D^{23}$= -70.2° (C=0.97, $CH_3CH_2OH$); 'H-NMR (δ): 1.17-1.40 (6H, m),

3.87-4.4 (6H, m), 4.87-5.0 (1H, dd), 5.17 (1H, s), 7.26 (5H, s), 7.47-7.60 (5H, m); IR (KBr): 1730-1790 $cm^{-1}$ (br, C=0).

Analysis calculated for $C_{25}H_{24}N_2O_7$:

C, 64.65; H, 5.17; N, 6.03.

Found: C, 65.28; H, 5.35; N, 6.07.

The 3-amino title compound was prepared by catalytic hydrogenation of 324 mg (0.72 mmole) of the Ox protected 3-amino β-lactam in 15 mL of ethanol containing 0.6 mL of 1.2 N HCl over 80 mg of 10% Pd/C at 35 psi for 4.5 hours. The mixture was then filtered through Celite. The Celite was washed with 50 mL of ethanol and the combined filtrate was evaporated. The residue was recrystallized from ethanol-ether to give 165.4 mg (0.59 mmole, 82%) of the title compound. M.P. 137-141° (d); $[\alpha]_D^{23}$ = -33.8 (C=0.4, ethanol; 'H-NMR (δ): 1.16-1.37 (6H, t), 3.93-4.4 (6H, m), 4.67-4.9 (1H, m), 5.3 (1H, s), 8-10 (3H, br); IR (KBr): 1780-1730 $cm^{-1}$ (br C=0).

Analysis calculated for $C_{10}H_{17}N_2O_5Cl$:

C, 42.86; H, 6.07; N, 10.0.

Found: C, 42.88; H, 6.16; N, 10.07.

## Example 9

1-(Diethoxycarbonylmethyl)-3-amino-4-methyl-2-azetidinone

L-Threonine (5 g, 42 mmoles) was mixed with 18 mL of benzyltriethylammonium hydroxide (40% in

methanol). After evaporating three times from ethanol (100 mL), the resulting oil was taken up in DMF (50 mL) and treated with 1,2-diphenylvinylene carbonate (10 g) for 4 hours. The mixture was acidified with 1.2 N HCl (40 mL) and extracted with ethyl acetate (200 mL). The organić layer was washed with water (3 x 100 mL), brine (100 mL), and dried over $MgSO_4$. The residue left after evaporation was taken up in 40 mL of TFA and stirred for 3 hours. The TFA was removed *in vacuo* and the residue was vacuum desiccated for 12 hours. The crude product was taken up in ethyl acetate (200 mL) and washed with water (5 x 50 mL), brine (100 mL), and dried over $Na_2SO_4$. After reducing the volume of ethyl acetate to 1/2, 100 mole % of dicyclohexylamine (DCHA) was added. On standing, the Ox-protected threonine DCHA salt crystallized producing 13.8 g (26.5 mmoles, 63%) in three crops. M.P. 190-192°C (ethanol-ether). NMR ($CDCl_3$/DMSO-$d_6$) (δ): 0.99-2.16 (23H, m); 2.7-3.2 (2H, br m); 3.7-3.76 (1H, d, J=6 Hz); 4.27-4.5 (2H, br m); 7.2 (5H, s); 7.3-7.6 (5H, br m).

Ox-L-Threonine DCHA salt (2 g, 3.85 mmoles), N-hydroxybenzotriazole (.57 g, 4.3 mmoles) and diethylaminomalonate hydrochloride (.9 g, 4.2 mmoles) were dissolved in 50 mL of methylene chloride. 1-Cyclohexyl-3-(2-morpholinoethyl)carbodiimide metho-p-toluenesulfonate (1.8 g, 4.3 mmoles) was added and the reaction stirred for 6 hours at R.T. On diluting with 100 ml. of ethyl acetate, the mixture was washed with 0.18M $H_2SO_4$ (2 x 50 mL), 10% $Na_2CO_3$ (3 x 25 mL), brine (50 mI), and dried over $MgSO_4$. The residue obtained after evaporation was chromatographed (silica, 9:1 methylene chloride to ether), yielding 0.9137 g (1.84 mmoles, 47%) of the N-diethoxycarbonylmethylamide. NMR ($CDCl_3$) (δ): 1.06-1.37 (9H, m); 3.87-3.93 (1H, d, J= 6Hz); 4.13-4.37 (4H, q); 3.67-4.77 (2H, br m); 5.07-5.13 (1H, d); 7.23 (5H, s); 7.33-7.5 (5H, br m); 8.1-8.25 (1H, br d).

Ox-L-Threonylaminomalonate (.45 g, .92 mmoles) and TPP (.26 g, .1 mmoles) were dissolved in THF (15 mL). DIAD (.196 mL, .1 mmoles) in THF (15 mL) was added dropwise over .5 hour, after which the reaction mixture was evaporated and chromatographed (silica, 9:1 methylene chloride to ether). A mixture (0.32 g) containing the product was eluted. By NMR this mixture contained the desired β-lactam along with the dehydropeptide and the pyrrolidinone. The ratio of products (73%) was roughly 2:1:1.- Rechromatography led to the isolation of the β-lactam (50 mg, .1 mmoles, 11%); M.P. 140-142°C. NMR ($CDCl_3$) (δ): 1.16-1.14 (9H, m); 4.09-4.5 (7H, m); 5.13 (1H, s); 7.23 (5H, s); 7.3-7.66 (5H, m).

The Ox protecting group is removed by catalytic hydrogenolysis of the cyclized product in ethyl alcohol using 10% palladium on carbon under about 35 psi of hydrogen to provide diethyl-3(S)-amino-1-malonyl-4-methyl-2-azetidinone represented by the formula

## Example 10

N-[(Diethoxycarbonyl)methyl]-3-[(R)-(tert-butyloxycarbonylamino]-4-(R)-methoxycarbonyl]-2-azetidinone.

L-Erythro-β-hydroxyaspartic acid (1 g., 6.7 mmoles) was dissolved in 15 ml. of methyl alcohol containing 1.1 ml. of concentrated hydrochloric acid and the solution was heated at the reflux temperature for 3 hours. On cooling, the reaction mixture was evaporated to dryness and the residue desiccated in vacuo. The solid product was triturated with diethyl ether and filtered to give 1.32 g (99%) of methyl β-hydroxyaspartic acid hydrochloride melting at about 182°C. to about 183°C.

The ester hydrochloride (600 mg., 3.0 mmoles) was dissolved in 15 ml. of THF-water (1-1) containing 660 mg. (3.0 mmoles) of t-butylpyrocarbonate and 1.25 ml. (9.0 mmoles) of triethylamine. After 1.5 hours the reaction mixture was extracted with 25 ml. of ethyl acetate and then acidified to pH 2 with cold 6N hydrochloric acid. The acidified mixture was extracted further with three 50 ml. portions of ethyl acetate. The extracts were combined, washed with 50 ml. of brine, dried over magnesium sulfate, filtered, and concentrated by evaporation to give 632 mg (2.4 mmoles, 80%) of methyl N-BOC-L-erythro-β-hydroxyaspartic acid. NMR ($CDCl_3$) (δ): 1.46 (s, 9H), 3.8 (s, 3H), 4.53 (d, 1H), 4.8-4.9 (br d, 1H), 5.73-5.8 (br d, 1H), and 6.9-7.1 (br s, 2H).

The t-BOC protected ester is converted (method B) to the amide formed with diethylaminomalonate and is represented by the formula

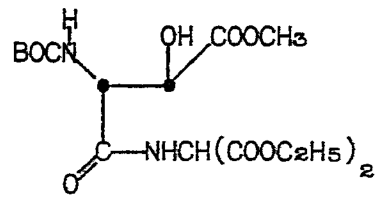

The amide is reacted with TPP and diisopropylazodicarboxylate to provide the title compound.

Example 11

N-(Sulfomethyl)-3-(4,5-diphenyl-2-oxo-4-oxazolino)-2-azetidinone.

Ox-protected-2-serine is coupled with aminomethanesulfonic acid tetra-n-butylammonium salt in tetrahydrofuran to provide the amide represented by the following formula:

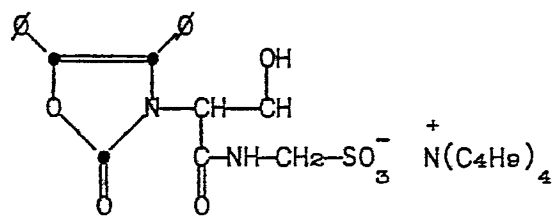

The amide is cyclized with TPP and diisopropylazodicarboxylate to provide the 3-(Ox-protected)-N-(sulfomethyl)-2-azetidinone.

CLAIMS

1. A compound of Formula (I):

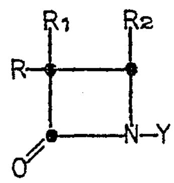 (I)

in which R is hydrogen, $C_1$-$C_4$ alkoxy, amino, protected-amino, acylamino, diacylamino, $C_1$-$C_4$ alkyl, or $C_1$-$C_4$ alkyl substituted by hydroxy, $C_1$-$C_4$ alkoxy, halogen, amino, protected-amino, carboxy, or protected carboxy;

$R_1$ is hydrogen, hydroxy, $C_1$-$C_4$ alkoxy, halogen, or $C_1$-$C_4$ alkyl;

$R_2$ is hydrogen, phenyl, substituted phenyl, carboxy, $C_1$-$C_4$ alkoxycarbonyl, protected carboxy, sulfydryl, $C_1$-$C_4$ alkylthio, $C_2$-$C_4$ alkanoylthio, halogen, $C_1$-$C_4$ alkoxy, $C_1$-$C_4$ alkyl, or $C_1$-$C_4$ alkyl substituted by hydroxy, $C_1$-$C_4$ alkoxy, halogen, amino, protected amino, carboxy, or protected carboxy;

Y is a substituted methyl group of the formulae

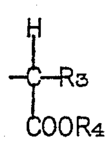 , 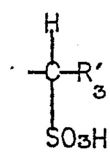 , or 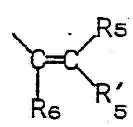

in which $R_3$ or $R_3'$ is hydrogen, $C_1$-$C_4$ alkoxycarbonyl, protected-carboxy, carboxy, $C_1$-$C_4$ alkylsulfonyl, arylsulfonyl, $C_1$-$C_4$ alkylsulfinyl, arylsulfinyl, $C_1$-$C_4$ alkanoyl, aroyl, $C_1$-$C_4$ alkyl, cyano, vinyl, or ethinyl;

$R_4$ is hydrogen, $C_1$-$C_4$ alkyl, or a carboxy protecting group;

$R_5$ and $R_5'$, independently are $C_1$-$C_4$ alkyl, $C_1$-$C_4$ alkoxycarbonyl, carboxy, protected-carboxy, or phenyl; and,

$R_6$ is hydrogen, $C_1$-$C_4$ alkyl, $C_1$-$C_4$ alkoxycarbonyl, protected-carboxy, or phenyl; or an addition salt thereof.

2. A compound as claimed in claim 1 in which R is an acylamino group.

3. A compound as claimed in claim 1 or 2 in which the acyl radical of the acylamino group is $C_1$-$C_5$ alkanoyl, $C_1$-$C_5$ alkanoyl substituted by halogen, cyano, or hydroxy; an arylacetyl or heteroarylacetyl group of the Formula

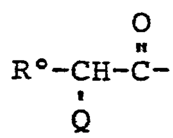

in which R° is thienyl, benzothienyl, furyl, benzofuryl, thiazolyl, oxazolyl, isoxazolyl, isothiazolyl, pyrazolyl, thiadiazolyl, oxadiazolyl, pyridyl, or said heterocyclic rings substituted by $C_1$-$C_4$ alkyl, amino, protected amino, or hydroxy; cyclohexadienyl, naphthyl, phenyl, or a substituted phenyl group of the Formula

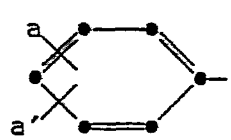

in which a and a', independently, are hydrogen, halogen, hydroxy, $C_1$-$C_4$ alkyl, $C_1$-$C_4$ alkoxy, amino, aminomethyl, methylsulfonylamino, hydroxymethyl, trifluoromethyl, carboxy, protected carboxy, carboxymethyl, or protected carboxymethyl;

Q is hydrogen, hydroxy, $C_1$-$C_4$ alkanoyloxy, carboxy, protected carboxy, sulfo ($-SO_3H$), amino, protected amino, or a substituted amino group of the Formula

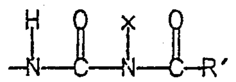

in which R' is furyl, thienyl, phenyl, halophenyl, nitrophenyl, styryl, halostyryl, nitrostyryl, or a group of the Formula

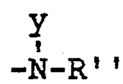

in which R'' is hydrogen, $C_1$-$C_4$ alkyl, benzyl, $C_2$-$C_5$ alkanoyl, or $C_1$-$C_3$ alkylsulfonyl; x and y, when taken separately, are hydrogen or $C_1$-$C_4$ alkyl, and when taken together form a 5- or 6-membered ring of the Formula

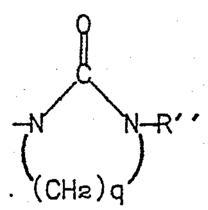

in which R'' has the same meanings as defined above and q is 2 or 3;

or Q is a substituted amino group of the Formula

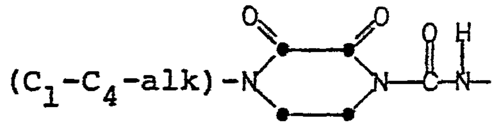

;

or Q is a benzamido group of the Formula

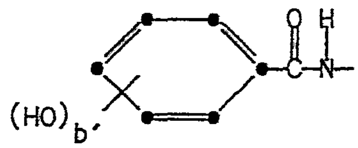

in which b' is an integer of 1-3; or R is an acylamino group in which the acyl moiety is a group of the formula

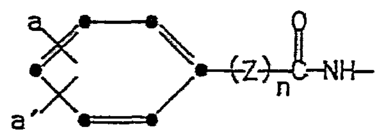

in which a and a' have the same meanings as defined earlier, Z is O or S, and n is 0 or 1; or an oximino-substituted acylamino group of the formula

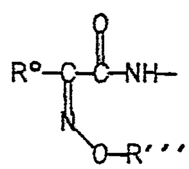

in which R° is as defined earlier, and R''' is hydrogen, $C_1$-$C_4$ alkyl, or a carboxy-substituted alkyl or carboxy-substituted cycloalkyl group of Formula

$$-\underset{d}{\overset{c}{C}}-(CH_2)_m-COOR''''$$

in which m is 0 to 3, and c and d when taken separately, are, independently, hydrogen or $C_1$-$C_3$ alkyl, and when taken together with the carbon atom to which they are bonded form a 3- to 6-membered carbocyclic ring; and R'''' is hydrogen, $C_1$-$C_4$ alkyl, or a carboxy-protecting ester group.

4. A compound of Formula (I) as claimed in any one of claims 1 to 3 in which R is phthalimido, $R^1$ and $R^2$ are hydrogen, and Y is $-CH_2COOMe$; or R is phthalimido, $R^1$ and $R^2$ are hydrogen and Y is $-CH(COOEt)_2$; or R is benzyloxycarbonylamino, $R^1$ and $R^2$ are hydrogen and Y is $-CH(COOEt)_2$; or R and $R^1$ are methyl, $R^2$ is hydrogen and Y is $-CH(COOEt)_2$; or R and $R^1$ are methyl, $R^2$ is hydrogen and Y is 3-ethoxycarbonylpropen-2-yl; or R and $R^1$ are methyl, $R^2$ is hydrogen and Y is 2-methyl-1-ethoxycarbonylpropen-1-yl; or R is amino, $R^1$ and $R^2$ are hydrogen and Y is $-CH(COOEt)_2$; or R is amino, $R^1$ is hydrogen, $R^2$ is methyl and Y is $-CH(COOEt)_2$; or R is t-butoxycarbonylamino, $R^1$ is H, $R^2$ is $-COOCH_3$ and Y is $-CH(COOEt)_2$; or R is diphenyl-2-oxo-4-oxazolino, $R^1$ and $R^2$ are H and Y is $-CH_2SO_3H$; or an unprotected derivative and/or addition salt thereof.

5. A process for preparing a compound of Formula (I) as defined in any one of claims 1 to 4 which comprises ring-closure of a compound of Formula (II)

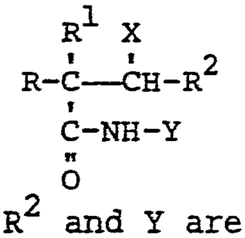

(II)

in which R, $R^1$, $R^2$ and Y are as defined in any one of claims 1 to 4, and X is hydroxy or halo.

6. A process as claimed in claim 5 in which ring closure is effected with a dialkylazodicarboxylate and an organo-phosphorous compound when X is hydroxy or with a lithium dialkylamide when X is halo.

7. A process as claimed in claim 6 in which the organo-phosphorous compound and the dialkylazodicarboxylate each are present in at least about 100 mole percent quantities when X is hydroxy or the dialkylamide is present in about 200 mole percent quantities when X is halo.

8. A process as claimed in claim 6 or 7 in which the dialkylazodicarboxylate is diisopropylazodicarboxylate or diethylazodicarboxylate and the organo-phosphorous compound is triphenylphosphine, triphenylphosphite, diphenyl phenylphosphate or phenyl diphenylphosphinoate.

9. A process as claimed in claim 6 or 7 in which the lithium dialkylamide is lithium diisopropylamide.

10. A compound of Formula (I) as claimed in any one of claims 1 to 4 for use in the production of antibiotics.